# EUROPÄISCHE PATENTSCHRIFT

(11) **EP 1 616 588 B1**
(45) Veröffentlichungstag und Bekanntmachung des Hinweises auf die Patenterteilung: **29.03.2006**
(21) Anmeldenummer: 04016909.6
(22) Anmeldetag: 17.07.2004
(51) Int. Cl.: A61M 5/142, A61M 39/28

(54) **Anordnung zum Kuppeln eines Intravenös-Schlauches mit einer Infusionspumpe**
Arrangement for the coupling of a intravenous tube with infusion pump
Système de couplage d'un tube intraveineux avec une pompe à perfusion

(43) Veröffentlichungstag der Anmeldung: 18.01.2006
(73) Patentinhaber: Codan Holding GmbH, 23738 Lensahn (DE)
(72) Erfinder: Hasler, Roland, 20022 Bevaix (CH)
(74) Vertreter: Fleck, Thomas

(56) Entgegenhaltungen:
- EP-B- 0 238 227
- WO-A-96/30679
- US-A1- 2002 161 333
- US-A1- 2002 165 503
- US-B1- 6 261 262

## Beschreibung

Die Erfindung betrifft eine Anordnung nach dem Oberbegriff des Anspruches 1.

Eine derartige Anordnung ist aus der EP 238 227 B1 bekannt. Diese fordert jedoch ein Klemmstück mit tropfenförmiger Öffnung, durch das der Schlauch hindurch verläuft und durch eine Einrichtung in dieser tropfenförmigen Öffnung zwischen Offenstellung und Schließstellung hin und her bewegt wird. Außerdem muss eine Sperreinrichtung an der Pumpe vorgesehen werden, um das Klemmstück mit der Pumpe anfänglich in Schließstellung zu stellen. Bei dieser vorbekannten Einrichtung muss der Intravenös-Schlauch zunächst einmal in die tropfenförmige Öffnung eingefädelt werden, wozu der Schlauch an irgendeinem Ende vom Infusionsset gelöst werden muss. Auch kann der Intravenös-Schlauch ohne Schlauchklemme in das Gehäuse der Infusionspumpe eingesetzt und anschließend die Gehäusetür verschlossen werden. Für Dritte könnte so der Eindruck entstehen, dass diese Anordnung funktionstüchtig ist, wodurch dann eine Fehlbedienung hervorgerufen werden kann.

Ziel der Erfindung ist es, dem Anwender, wie Pflegern, Krankenschwestern und Ärzten, ein System bzw. eine Anordnung zur Verfügung zu stellen, bzw. die eingangs genannte Anordnung derart zu verbessern, dass eine unkontrollierte Abgabe von Infusionslösungen jeglicher Art in den Patienten ausgeschlossen werden kann. Mit anderen Worten: Fehlbedienungen durch das Bedienungspersonal sollen möglichst mit Sicherheit verhindert werden.

Erfindungsgemäß wird dieses bei der eingangs genannten Anordnung dadurch erreicht, dass die Schlauchklemme relativ zueinander um ein Gelenk bewegbare Klemmflächen in Form von Schenkeln besitzt, die an ihren Außenseiten von einer vorgespannten Klemmfeder umgeben sind,
und dass die Einrichtung durch eine Spreizrippe gebildet wird, welche an der Innenseite der Tür befestigt ist und mit den Schenkeln der Schlauchklemme zur Herstellung der Offenstellung nach dem Schließen der Tür zum Spreizen derselben in Eingriff bringbar ist,
und dass das Gehäuse eine Aufnahme besitzt, in der die Schlauchklemme verrastbar ist,
und dass eine Sperreinrichtung im Gehäuse vorhanden ist, die bei nicht in die Aufnahme eingesetzter Schlauchklemme ein Schließen der Tür verhindert und bei vollständig eingesetzter Schlauchklemme die Tür zum Schließen freigibt.

Die erfindungsgemäße Anordnung ist also einfach aufgebaut und lässt sich deshalb in wirtschaftlicher Weise fertigen, wobei gleichermaßen sichergestellt ist, dass ein Risikopotential durch Fehlbedienungen ausgeschlossen werden kann.

Weitere Vorteile und Merkmale gehen aus den Unteransprüchen hervor, die auch gemeinsam mit dem Hauptanspruch von erfinderischer Bedeutung sein können.

Im Folgenden wird ein bevorzugtes Ausführungsbeispiel anhand der Zeichnung näher erläutert, um ein besseres Verständnis der Erfindung zu erzielen. Es dürfte jedoch einleuchten, dass die Erfindung nicht auf dieses Ausführungsbeispiel beschränkt ist.

Es zeigt:
- Fig. 1: eine perspektivische Darstellung der erfindungsgemäßen Anordnung;
- Fig. 1A: eine perspektivische Darstellung der erfindungsgemäßen Anordnung mit geöffneter Tür der Infusionspumpe vor der Inbetriebnahme;
- Fig. 1B: eine schematische Perspektivdarstellung im vergrößerten Ausschnitt aus Fig. 1;
- Fig. 2: eine weitere schematische Perspektivdarstellung im vergrößerten Ausschnitt aus Fig. 1;
- Fig. 3: eine weitere perspektivische schematische Darstellung der Aufnahme mit dem Verriegelungsschieber im Ausschnitt vom Inneren der Pumpe her;

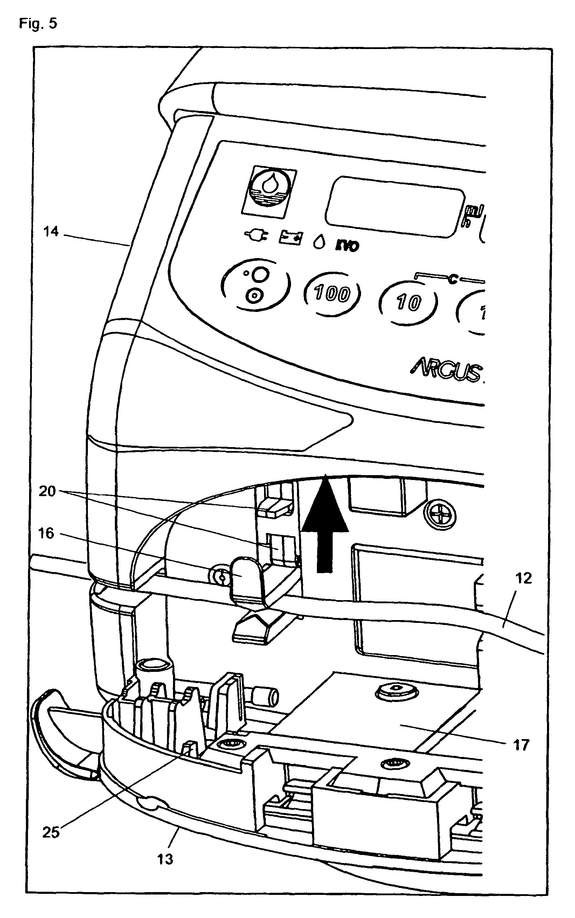

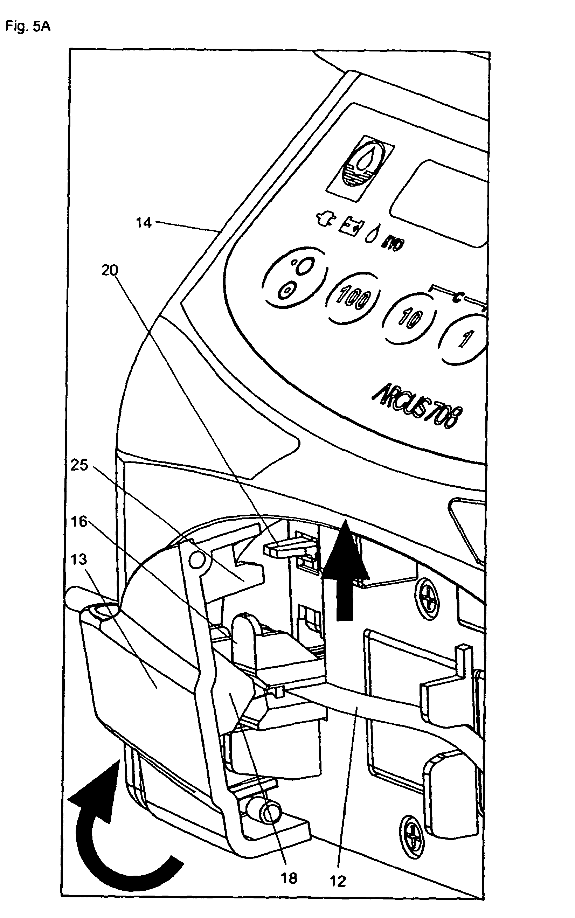

- Fig. 4: eine weitere schematische Perspektivdarstellung des federbelasteten Verriegelungsschiebers;
- Fig. 5: eine schematische Ausschnittsansicht ähnlich zu Fig. 2, jedoch mit in der Aufnahme verrasteten Schlauchklemme bei geöffneter Tür;
- Fig. 5A: eine weitere schematische Perspektivansicht im Ausschnitt, bei der Teile weggebrochen sind und die Tür hochgeklappt wird, wobei der Verriegelungsschieber noch nicht auf der Anschlagrippe liegt;
- Fig. 5B: eine schematische Ansicht, ähnlich zur Fig. 5A, wobei jedoch die Tür geschlossen und die Schenkel der Schlauchklemme durch die Spreizrippe auseinander gedrückt werden, so dass der Schlauch für den Durchfluss frei wird;
- Fig. 5C: eine weitere schematische Perspektivdarstellung im Ausschnitt, bei der die Tür weggebrochen im Öffnungsvorgang dargestellt wird;
- Fig. 6: eine Perspektivdarstellung der erfindungsgemäßen Anordnung mit eingesetztem Schlauch ohne Schlauchklemme, so dass die Tür nicht vollständig geschlossen ist;
- Fig. 6A: eine schematische Perspektivdarstellung im Ausschnitt mit teilweise weggebrochener Tür, die aufgrund des gegen den Anschlag drückenden Verriegelungsschiebers nicht geschlossen ist;
- Fig. 7: eine schematische Perspektivdarstellung eines Teiles des Pumpengehäuses mit Sicherungsmagneten;
- Fig. 8: eine schematische Perspektivdarstellung der erfindungsgemäß verwendeten Schlauchklemme mit Stahlbiegefeder; und
- Fig. 9: eine schematische Perspektivdarstellung der Aufnahme bzw. des Einschubmoduls mit Leiterplatte und Sensor.

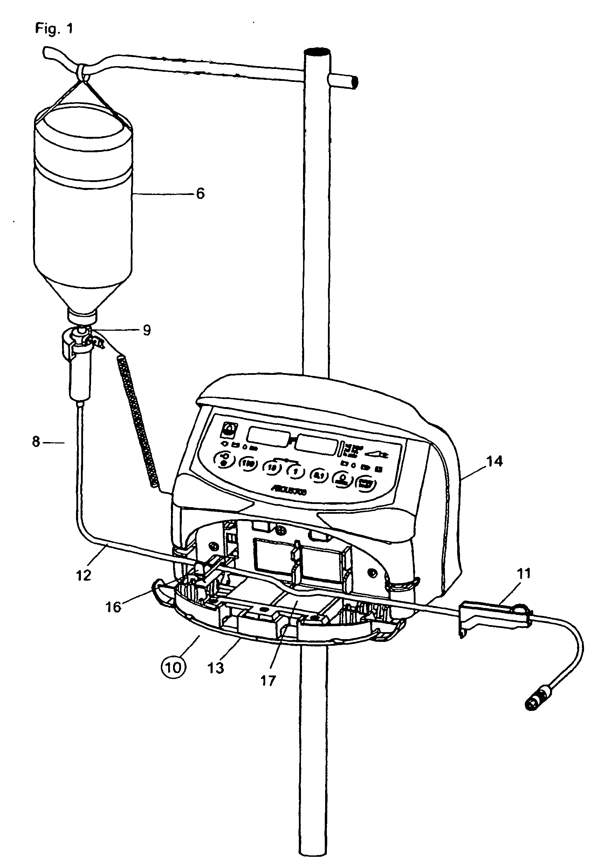

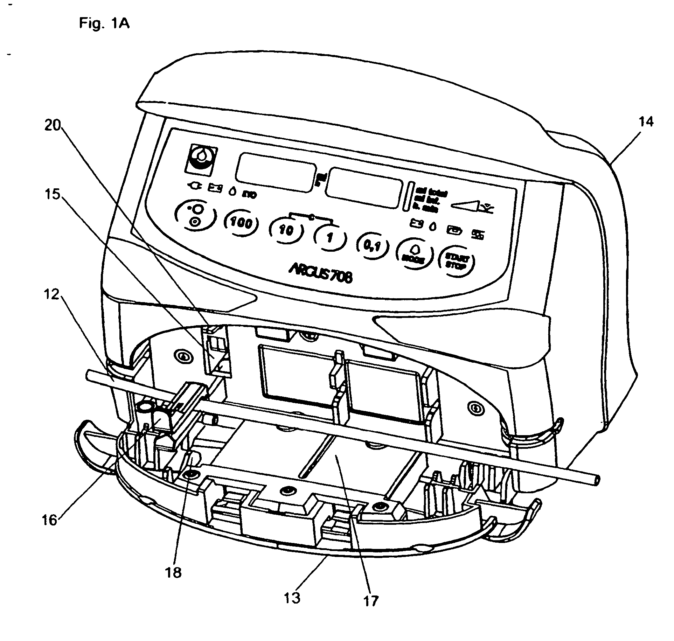

In Fig. 1 ist die erfindungsgemäße Anordnung allgemein mit 10 bezeichnet. Sie besteht aus üblichen Infusionsset 8 mit einer einen Einstechdorn aufweisenden Tropfenkammer 9, der in einem die Medikamentenlösung enthaltenden Infusionsbehälter 6 eingestochen ist, und einer Schlauchklemme 11 für den Schlauch 12 und einer Infusionspumpe 14, die in einem Gehäuse angeordnet ist.

Die Rollenklemme 11 hat bei Verwendung von volumetrischen Infusionspumpen 14 nur noch die Funktion zu erfüllen, den Schlauch 12 nach erfolgter Zubereitung der Infusion (Verbinden des Infusionssets mit dem Infusionsbehältnis, Füllen und Entlüften des Sets) abzuklemmen, um ein Auslaufen der Flüssigkeit zu verhindern, bis dass der Schlauch 12 im Antrieb der Pumpe 14 fixiert ist.

Nach dem Einlegen des Schlauches 12 in die Pumpe und dem Schließen der Pumpentür 13 wird der Schlauch 12 durch die Kombination aus peristaltischem Antrieb und Andruckplatte 17 abgeklemmt. Anschließend wird die Rollenklemme 11 des Infusionssets geöffnet, und das System bzw. die Anordnung 10 dahingehend überprüft, ob die Pumpe 14 den Schlauch 12 vollständig abgeklemmt.

Während des Förderbetriebes der Pumpe 14 ist die Rollenklemme 11 somit vollständig geöffnet. Öffnet nun während des Betriebes der Pumpe 14 eine Person die Pumpentür 13, ohne vorher die Rollenklemme 11 zu verschließen, besteht die Gefahr, dass Infusionsflüssigkeit unkontrolliert zum Patienten gelangt. Um dieses zu verhindern, ist die erfindungsgemäße Pumpe 14 mit einer sog. Anti-Free-Flow Schlauchklemme 16 versehen. Nach dem Öffnen der Pumpentür 13 klemmt dabei eine Sperrvorrichtung den Intravenös-Schlauch ab, um den Fluss zu verhindern.

Anschließend muss der Anwender die Rollenklemme 11 schließen, bevor der Schlauch 12 aus der Pumpe 14 entnommen wird.

An dieser Stelle zeigt sich - wie schon eingangs erwähnt - das Gefahrenpotential einiger bestehender Anordnungen und Systeme, da der Schlauch 12 nach erfolgter Entnahme aus der Pumpe 14 nur dann weiterhin verschlossen ist, wenn der Anwender wie bereits beschrieben die Rollenklemme 11 auf dem Infusionsset 8 verschlossen hat. Hat der Anwender die Klemme 11 jedoch nicht verschlossen, und wird das Infusionsset 8 aus der Pumpe 14 entnommen, kommt es zu einem unkontrollierten Fließen von Infusionslösung zum Patienten. Somit kann es durch einen Fehler in der Bedienung von Infusionsset und Pumpe 14 zu einer erheblichen Patientengefährdung kommen.

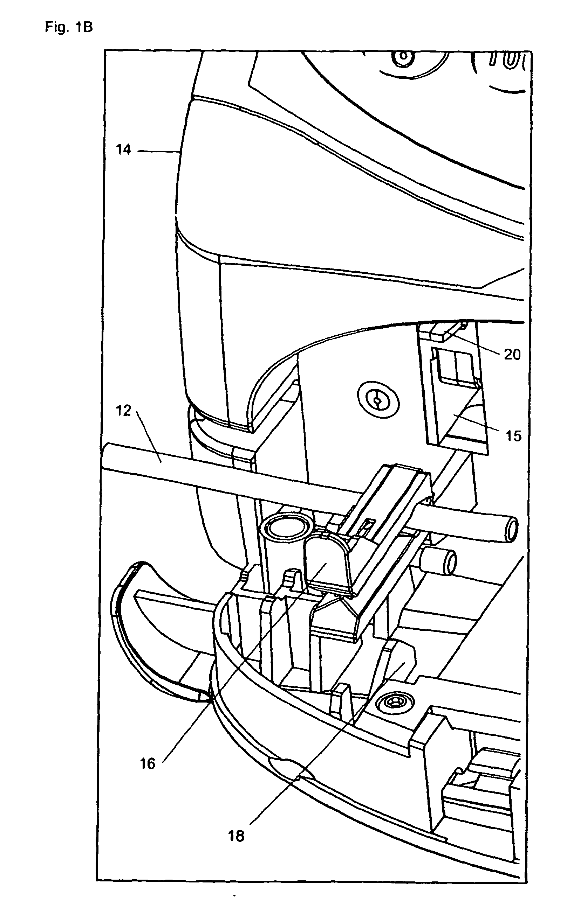

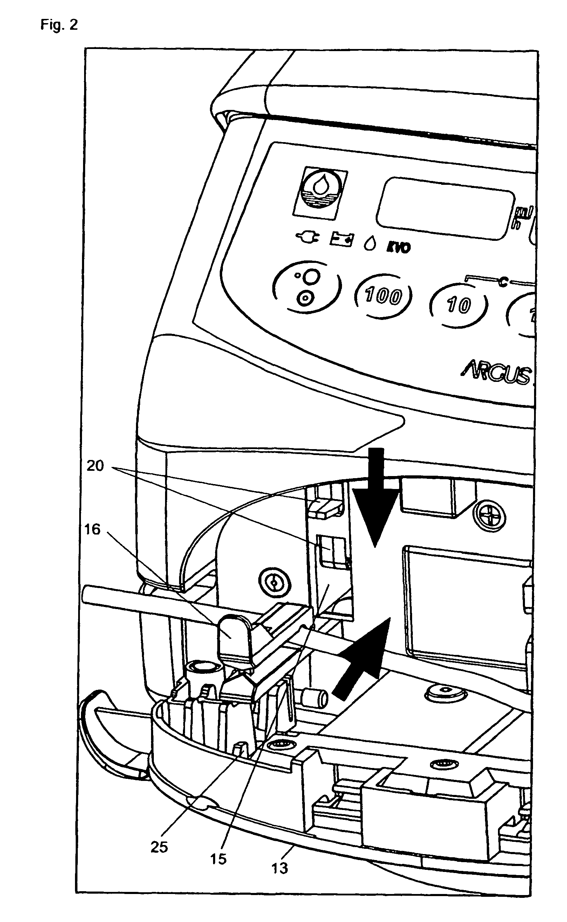

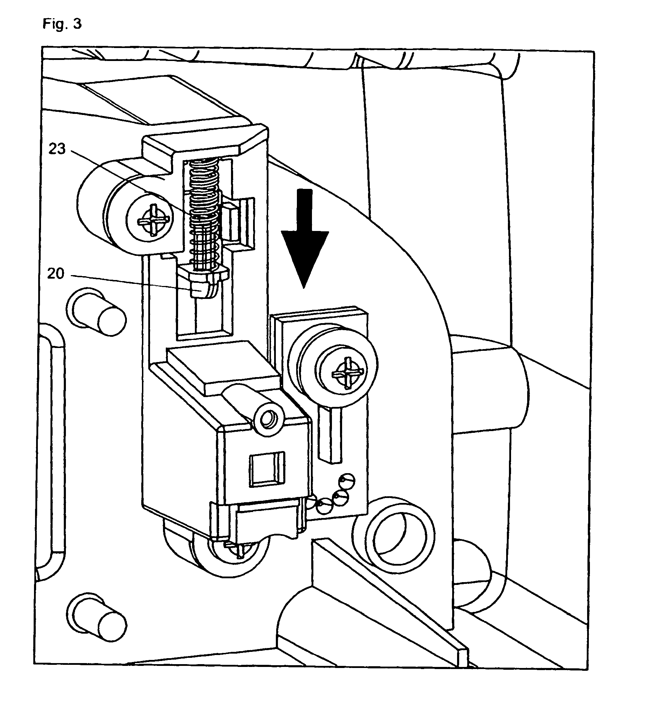

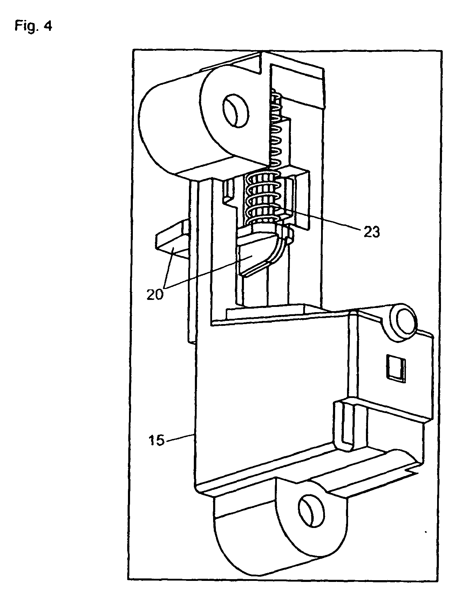

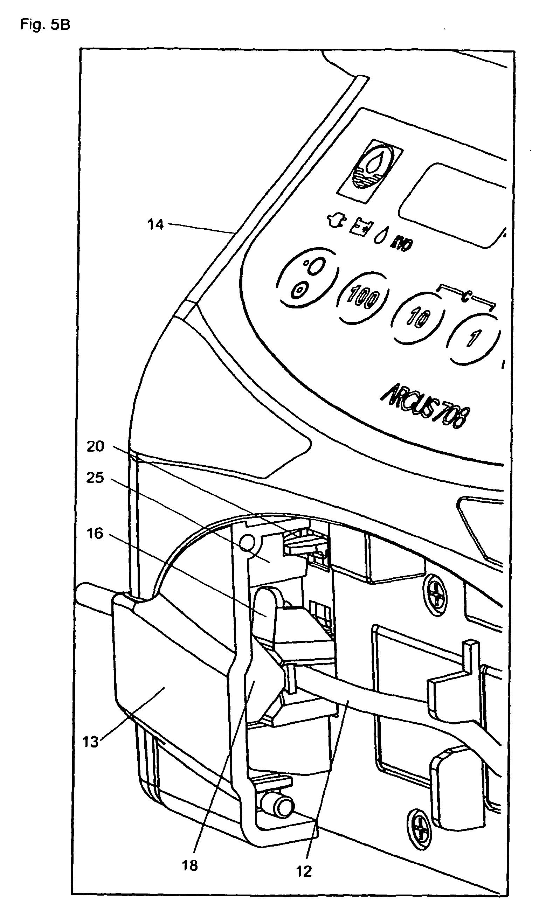

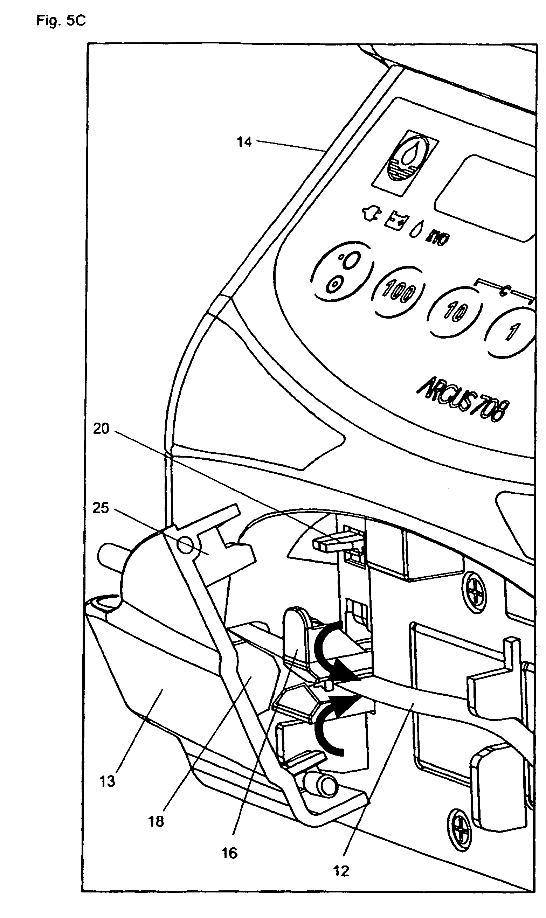

Zur Inbetriebnahme der erfindungsgemäßen Anordnung 10 werden folgende Schritte durchgeführt:
1. Vorbereitung des Infusionssets 8:
   ➢ Schließen der Rollenklemme 11
   ➢ Einstechen der Tropfkammer 9 in den Infusionsbehälter 6
   ➢ Füllen der Tropfkammer 9
   ➢ Öffnen der Rollenklemme 11 und Entlüften des Infusionssets
   ➢ Schließen der Rollenklemme 11
2. Öffnen der Pumpentür 13
3. Horizontales Einlegen des Intravenös-Schlauches 12 in die Pumpe 14 (siehe Fig. 1A und 1B). Einsetzen der SchlauchKlemme 16 in die dafür vorgesehene Aufnahme 15 in der Pumpe. Vor dem Einlegen der Schlauchklemme 16 in die Aufnahme 15 der Pumpe 14 ist die Klemme 16 geöffnet, d.h. der Schlauch 12 ist zu diesem Zeitpunkt nur durch die geschlossene Rollenklemme 11 abgeklemmt, die Schlauchklemme 16 ist jedoch noch geöffnet und somit in Schlauchrichtung auf dem Schlauch 12 in der Position veränderbar.
   Die als Verriegelungsschieber 20 ausgebildete Sperreinrichtung der Pumpe 14 ist ohne eingesetzte Klemme 16 durch eine Feder 23 belastet auf einen unteren Anschlag, der Pumpe 14 gedrückt (siehe Fig. 2 und 3).
   Durch das Einschieben der Schlauchklemme 16 in die Aufnahme 15 der Pumpe 14 wird der Verriegelungsschieber 20 nach oben in Pfeilrichtung gedrückt und behält diese Position (siehe Fig. 5 + 5A). Gleichzeitig wird der Schlauch 12 in der Pumpe 14 fixiert und die Schlauchklemme 16 nach hinten in die Aufnahme 15 geschoben. Dadurch wird die Schlauchklemme 16 in den Bereich über den Schlauch 12 geschoben, in dem die Schlauchklemme 16 den Schlauch 12 abklemmt.
   Das Schließen der Pumpentür 13 wird in den Fig. 5A und B gezeigt. Die Pumpentür 13 kann erst dann geschlossen werden, wenn die Schlauchklemme 16 in die Aufnahme eingesetzt wurde und somit der Verriegelungsschieber 20 in seine obere Lage gedrückt wurde.
   In Fig. 5A ist, gezeigt, dass die Tür 13 nun geschlossen werden kann, da sie nicht mehr durch den gegen den Anschlag 25 anliegenden Verriegelungsschieber 20 blockiert wird.
4. Zum Betrieb der Pumpe 14 ist es notwendig, die Schlauchklemme 16 zu öffnen, da der Schlauch 12 bei geschlossener Tür 13 durch die Klemmung zwischen einer Einrichtung 17 in Form einer Andruckplatte der Pumpentür 13 und peristaltischem Antrieb abgeklemmt wird, was dadurch erreicht wird, dass die Schlauchklemme 16 durch eine in der Tür 13 befindliche Spreizrippe 18 nach dem vollständigen Verschließen der Pumpentür 13 geöffnet wird, wie dieses durch die Pfeile angedeutet wird (siehe Fig. 5A + 5B).
5. Nach Schließen der Pumpentür 13 kann nun die Rollenklemme 11 auf dem Infusionsset 8 geöffnet werden, und das Set hinsichtlich eines vollständigen Abklemmens des Schlauches 12 durch die Peristaltik der Pumpe 14 überprüft werden (es darf kein Fließen der Infusionslösung erfolgen). Die Pumpe 14 kann gestartet werden.

Zum sachgemäßen Beenden des Betriebes der Pumpe 14 wird folgendermaßen vorgegangen:
1. Schließen der Rollenklemme 11
2. Öffnen der Pumpentür 13
   Durch das Öffnen der Pumpentür 13 schließt sich die Schlauchklemme 16 automatisch, da die Spreizrippe 18 der Tür 13 die Klemme 16 nicht mehr offen hält. Somit klemmt die Schlauchklemme 16 den Schlauch 12 durch den Druck der auf der Schlauchklemme 16 montierten Stahlfeder 21 wieder selbsttätig ab, wie dieses durch die Pfeile in Fig. 5C angedeutet wird.
3. Entfernen des Schlauches 12 mit Schlauchklemme 16 aus der Pumpe 14.
4. Nach dem Öffnen der Tür 13 kann der Schlauch 12 mit Klemme 16 und Feder 21 aus der Pumpe 14 entnommen werden. Die Schlauchklemme 16 mit Feder 21 wird aus der Aufnahme 15 entfernt, wobei der Verriegelungsschieber 20 durch den Federdruck in seine Ausgangsposition zurückgedrückt wird (Fig. 2).

Somit ist die erfindungsgemäße Anordnung 10 mit der Infusionspumpe 14 bei sachgemäßer Handhabung einerseits durch die manuell geschlossene Rollenklemme 11, und andererseits durch die automatisch abklemmende Schlauchklemme 16 gegen einen unkontrollierten Fluss oder sog. "free flow" von Infusionslösung zum Patienten gesichert.

Der Betrieb der Infusionspumpe 14 kann auf verschiedene Arten unsachgemäß beendet werden.

Wird z.B. die Pumpentür 13 geöffnet, ohne dass die Rollenklemme 11 auf dem Infusionsset 8 vorher vom Bediener manuell geschlossen wurde, verschließt die Schlauchklemme 16 den Schlauch 12 - wie vorher beschrieben - ebenfalls automatisch.

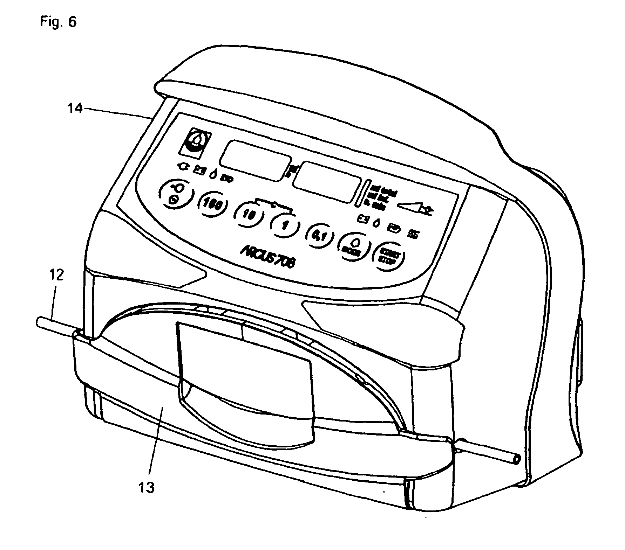

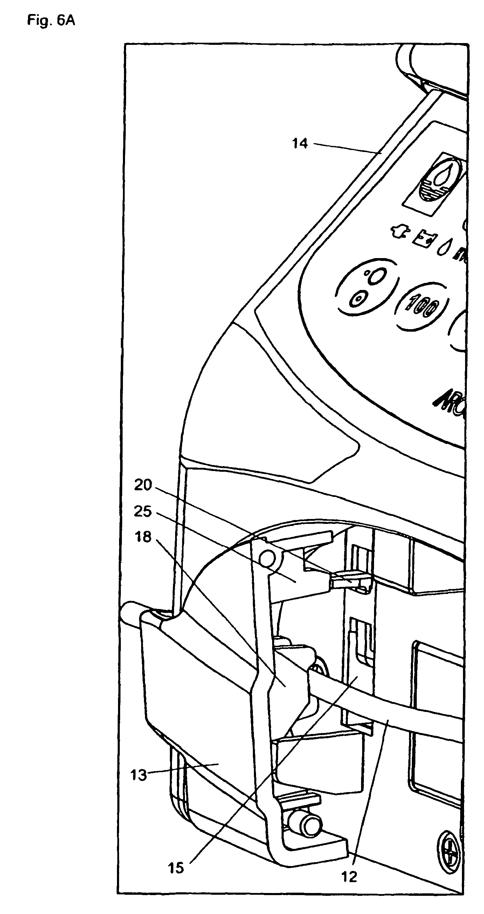

Eine weitere mögliche Ursache für Fehlbedienungen ist die Inbetriebnahme der Infusionspumpe 14 ohne die Schlauchklemme 16. Die entscheidende Verbesserung der erfindungsgemäßen Anordnung 10 besteht nun darin, dass die Infusionspumpe 14 ohne eine eingelegte Schlauchklemme 16 mit Feder 21 gar nicht erst in Betrieb genommen werden kann. Dieses wird dadurch sichergestellt, dass die Pumpentür 13 nicht vollständig verschlossen werden kann (siehe Fig. 6 + 6A), wenn die Schlauchklemme 16 mit Feder 21 nicht in die Aufnahme 15 eingelegt wurde.

Wird die Schlauchklemmeinheit 16/21 nicht in die Aufnahme 15 eingelegt, ist der Verriegelungsschieber 20 durch den Federdruck in die untere Endlage gedrückt (vgl. Pfeil in Fig. 2). Die Tür 13 kann nicht geschlossen werden, da die Anschlagrippe 25 an der Tür 13 genau die Position des Verriegelungsschiebers 20 hat, wenn keine Schlauchklemme 16 eingelegt ist (siehe Fig. 6A).

Dadurch kann die Pumpentür 13 nicht geschlossen werden, ein Betrieb der Pumpe 14 ist nicht möglich. Somit ist ein unbeabsichtigter Strom der Infusionslösung zum Patienten auszuschließen, da der Anwender die Pumpentür 13 nicht schließen kann, und demzufolge auch nicht die Rollenklemme 11, die nach Vorbereitung der Infusion geschlossen wurde, öffnen wird.

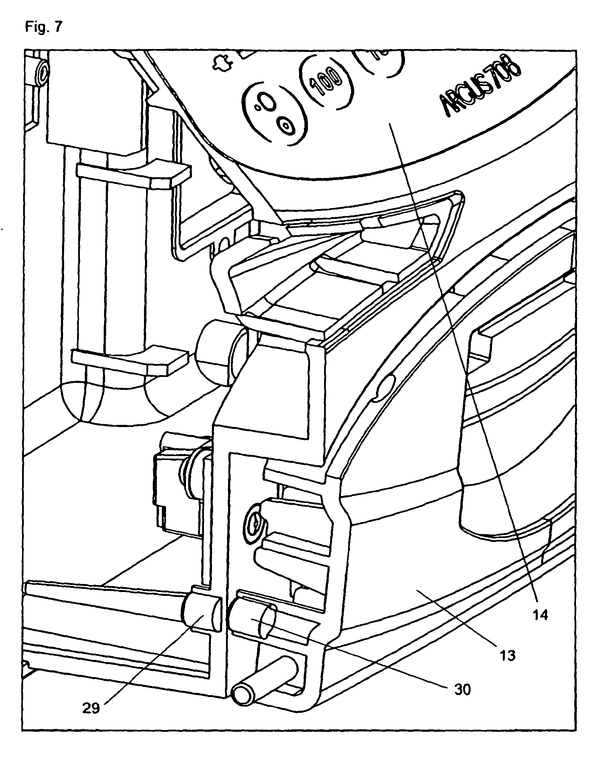

Damit die Pumpe 14 nicht mit offener Tür 13 gelagert werden muss, wird diese durch zwei Magnete in der Position gehalten, bei welcher die Anschlagrippe auf dem Verriegelungsschieber 20 ansteht. Während einer der Magnete 29, 30 in der unteren Ecke des Gehäuses angeordnet ist, ist der andere gegenüberliegend im Randbereich der Tür befestigt, wie es in Fig. 7 dargestellt ist.

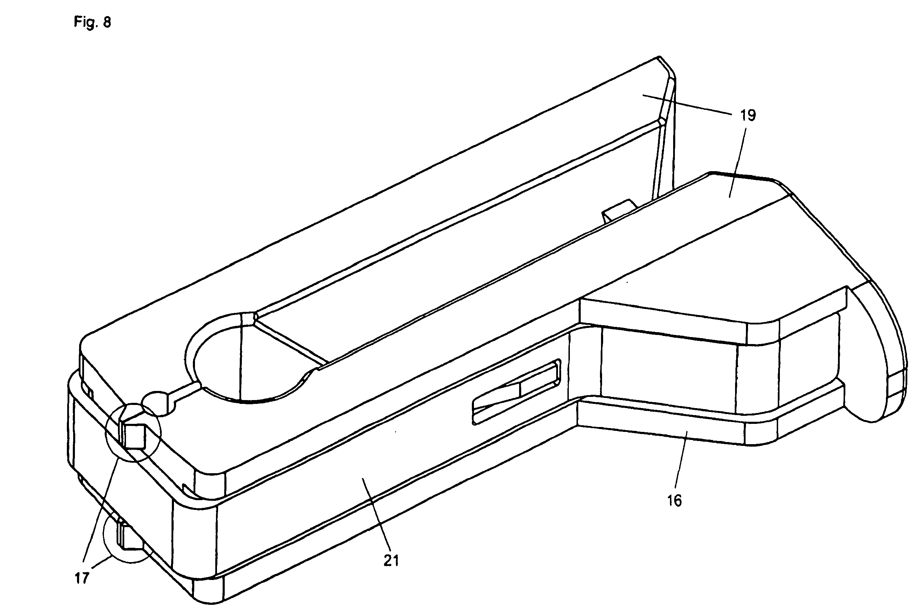

Zusätzlich ist die Schlauchklemme 16 des Ausführungsbeispiels, die relativ zueinander um ein Gelenk 17 bewegbare Schenkel 19 als Klemmflächen besitzt, unsymmetrisch konzipiert und ist an den Schenkelaußenseiten von einer Klemmfeder 21 umgeben (siehe Fig. 8). Dadurch ist es dem Anwender ebenfalls nicht möglich, das Infusionsset 8 falsch herum, d.h. entgegen der Pumprichtung einzulegen.

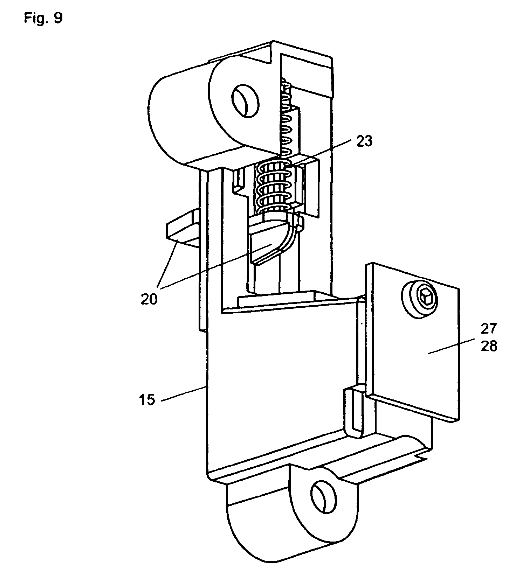

Als zusätzliche Sicherheit wird vorteilhafterweise an der Aufnahme 15, die man auch als Einschubmodul bezeichnen könnte, eine Leiterplatte 27 mit einem Sensor 28 montiert (vgl. Fig. 9, in der die Aufnahme isoliert dargestellt ist). Der Sensor 28 detektiert über die Stahlfeder 21 auf der Schlauchklemme 16, ob die Klemmeinheit 16/21 in die Aufnahme 15 eingeschoben wurde. Nur wenn der Sensor 28 "Schlauchklemme 16 eingelegt" als Signal weitergibt, kann die Pumpe 14 gestartet werden.

## Patentansprüche

1. Anordnung (10) zum Kuppeln eines eine Rollenklemme (11) aufweisenden Intravenös-Schlauches (12) mit einer Infusionspumpe (14) mit Gehäuse zur Abgabe einer medizinischen Lösung in einen Patienten, umfassend:
eine mit dem Intravenös-Schlauch (12) kuppelbare Schlauchklemme (16), die zwischen einer Offenstellung, in welcher die medizinische Lösung durch den Schlauch (12) strömen kann, und einer Schließstellung, in welcher der Schlauch (12) durch die Schlauchklemme (16) verschlossen ist, bewegbar ist,
eine an der Tür (13) oder Klappe des Gehäuses angebrachte Einrichtung (18), die mit der Schlauchklemme (16) in Eingriff bringbar ist, um dieselbe aus der Schließstellung in die Offenstellung zu bringen,
**dadurch gekennzeichnet, dass**
die Schlauchklemme (16) relativ zueinander um ein Gelenk (17) bewegbare Klemmflächen in Form von Schenkeln (19) besitzt, die an ihren Außenseiten von einer vorgespannten Klemmfeder (21) umgeben sind,
und dass die Einrichtung (18) durch eine Spreizrippe gebildet wird, welche an der Innenseite der Tür (13) befestigt ist und mit den Schenkeln (19) der Schlauchklemme (16) zur Herstellung der Offenstellung nach dem Schließen der Tür (13) zum Spreizen derselben in Eingriff bringbar ist,
und dass das Gehäuse eine Aufnahme (15) besitzt, in der die Schlauchklemme (16) verrastbar ist,
und dass eine Sperreinrichtung (20,25) im Gehäuse vorhanden ist, die bei nicht in die Aufnahme (15) eingesetzter Schlauchklemme (16) ein Schließen der Tür (13) verhindert und bei vollständig eingesetzter Schlauchklemme (16) die Tür (13) zum Schließen freigibt.

2. Anordnung (10) nach Anspruch 1, **dadurch gekennzeichnet, dass** die Sperreinrichtung durch einen mittels einer Feder (23) vorgespannten Verriegelungsschieber (20) gebildet wird, der mit einem Anschlag (25) auf der Innenseite der Tür (13) zusammenwirkt und auf einer schrägen Fläche desselben gleitet, wenn die Schlauchklemme (16) in die Infusionspumpe (14) zur Herstellung der Offenstellung eingesetzt und die Tür (13) geschlossen wird.

3. Anordnung (10) nach einem oder mehreren der vorstehenden Ansprüche, **dadurch gekennzeichnet, dass** im Bereich der Aufnahme (15) eine Leiterplatte (27) mit einem Sensor (28) angebracht wird, der detektiert, ob die Schlauchklemme eingeschoben ist oder nicht.

4. Anordnung (10) nach den Ansprüchen 1 bis 3, **dadurch gekennzeichnet, dass** zur Lagerung der Infusionspumpe (14) im Nichtgebrauchszustand eine Magnetsicherung (29,30) vorgesehen wird, die die Tür (13) geschlossen hält, während der Anschlag (25) auf dem Verriegelungsschieber (20) angeordnet ist.

5. Anordnung (10) nach Anspruch 1 bis 4, **dadurch gekennzeichnet, dass** die Schlauchklemme (16) formschlüssig in der Aufnahme (15) verrastbar ist.

6. Anordnung (10) nach Anspruch 1 bis 5, **dadurch gekennzeichnet, dass** im Gehäuse horizontale Schlauchführungen ein- und ausgangsseitig vorgesehen sind.

7. Anordnung (10) nach Anspruch 1 bis 6, **dadurch gekennzeichnet, dass** die Tür (13) über die fast gesamte Breite des Gehäuses verläuft und von unten nach oben in die Schließstellung verschwenkbar ist.

8. Anordnung (10) nach einem oder mehreren der Ansprüche 1 bis 7, **dadurch gekennzeichnet, dass** die anti-free-flow Schlauchklemme (16) nach dem Gehäuseeintritt und vor dem Antrieb der Infusionspumpe positioniert ist und aus einem Kunststoffspritzteil besteht, dessen Schenkel (19) von einer Metallklemmfeder (21) umgeben sind.

9. Anordnung (10) nach einem oder mehreren der vorstehenden Ansprüche 1 bis 8, **gekennzeichnet durch** einen PVC oder PVC-(freien) Klemmschlauch (12) mit einer Shorehärte A 75 bis 85, bei einem Innendurchmesser von 3,0 mm und einem Außendurchmesser von 4,10 mm, wobei die Klemmkraft der Schlauchklemme (16) min. 10N, insbesondere 15N beträgt.

10. Anordnung (10) nach einem oder mehreren der vorstehenden Ansprüche 1 bis 9, **gekennzeichnet durch** eine volumetrische Infusionspumpe (14) mit peristaltischem Antrieb.

## Claims

1. Arrangement (10) for coupling an intravenous tube (12) having a roller clamp (11) to an infusion pump (14) with a casing for delivering a medical solution to a patient, domprising:
a tube clamp (16) couplable to the intravenous tube (12) and which is movable between an open position in which the medical solution can flow through the tube (12) and a closed position in which the tube (12) is closed by a tube clamp (16),
a device (18) fitted to the door (13) or flap of the casing and which can be engaged with the tube clamp (16) in order to bring the latter from the closed position into the open position,
**characterized in that**
the tube clamp (16) has clamping surfaces in the form of legs (19) movable relative to one another about a joint (17) and which on their outsides are surrounded by a pretensioned clamping spring (21),
and the device (18) is formed by an expanding rib, which is fixed to the inside of the door (13) and can be engaged with the legs (19) of the tube clamp (16) for producing the open position after the closing of the door (13) in order to expand the same,
and the casing has a receptacle (15) in which the tube clamp (16) can be locked,
and a blocking device (20, 25) is provided in the casing for preventing a closing of the door (13) when the tube clamp (16) is not in the receptacle (15) and which releases the door (13) for closing purposes when the tube clamp (16) is fully inserted.

2. Arrangement (10) according to claim 1, **characterized in that** the blocking device is formed by a locking slide (20) pretensioned by means of a spring (23) and which cooperates with a stop (25) on the inside of the door (13) and slides on a sloping surface thereof if the tube clamp (16) is inserted in the infusion pump (14) for producing the open position and the door (13) is closed.

3. Arrangement (10) according to one or more of the preceding claims, **characterized in that** in the vicinity of the receptacle (15) is installed a printed circuit board (27) with a sensor (28) detecting whether or not the tube clamp has been slid in.

4. Arrangement (10) according to claims 1 to 3, **characterized in that** for mounting the infusion pump (14) in the non-use state a magnetic safety device (29, 30) is provided, which keeps the door (13) closed whilst the stop (25) is located on the locking slide (20).

5. Arrangement (10) according to claims 1 to 4, **characterized in that** the tube clamp (16) is positively lockable in the receptacle (15).

6. Arrangement (10) according to claims 1 to 5, **characterized in that** on the inlet and outlet sides of the casing horizontal tube guides are provided.

7. Arrangement (10) according to claims 1 to 6, **characterized in that** the door (13) passes over virtually the entire casing width and is pivotable from bottom to top into the closed position.

8. Arrangement (10) according to one or more of the claims 1 to 7, **characterized in that** the anti-free-flow tube clamp (16) is positioned downstream of the casing inlet and upstream of the infusion pump drive and comprises a plastics moulding, whose legs (19) are surrounded by a metal clamping spring (21).

9. Arrangement (10) according to one or more of the preceding claims 1 to 8, **characterized by** a PVC or PVC-free clamping tube (12) with a Shore A hardness of 75 to 85, an internal diameter of 3.0 mm and an external diameter of 4.10 mm, the clamping force of the tube clamp (16) being min. 10N and in particular 15N.

10. Arrangement (10) according to one or more of the preceding claims 1 to 9, **characterized by** a volumetric infusion pump (14) with a peristaltic drive.

## Revendications

1. Agencement (10) pour le couplage d'un tube intraveineux (12) présentant une pince à molette (11) avec une pompe à perfusion (14) avec boîtier pour administrer une solution médicale à un patient, comprenant :
une pince de tube (16) raccordable au tube intraveineux (12) qui peut être commutable d'une position d'ouverture dans laquelle la solution médicale peut s'écouler à travers le tube (12) à une position de fermeture dans laquelle le tube (12) est fermé par la pince de tube (16),
un dispositif (18) placé sur la porte (13) ou le couvercle du boîtier qui peut être amené en prise avec la pince de tube (16) afin d'amener celle-ci de la position de fermeture à la position d'ouverture,
**caractérisé en ce que** la pince de tube (16) possède des surfaces de pinces en forme de branches (19) relativement mobiles l'une par rapport à l'autre sur une articulation (17) qui sont entourées sur leurs faces externes par un ressort de friction (21) précontraint,
et **en ce que** le dispositif (18) est formé par une nervure d'écartement, laquelle est fixée sur la face interne de la porte (13) et qui peut être amenée en prise avec les branches (19) de la pince de tube (16) pour former la position d'ouverture après la fermeture de la porte (13) pour écarter celle-ci,
et **en ce que** le boîtier possède un logement (15) dans lequel la pince de tube (16) est encliquetable,
et **en ce qu'**un dispositif de blocage (20,25) est présent dans le boîtier qui empêche une fermeture de la porte (13) lorsque la pince de tube (16) n'est pas engagée dans le logement (15) et qui libère la porte (13) pour qu'elle se ferme lorsque la pince de tube (16) est totalement engagée.

2. Agencement (10) selon la revendication 1,
**caractérisé en ce que** le dispositif de blocage est formé par une targette de verrouillage (20) précontrainte au moyen d'un ressort (23), laquelle interagit avec une butée (25) sur la face interne de la porte (13) et glisse sur une surface inclinée de celle-ci lorsque la pince de tube (16) est engagée dans la pompe à perfusion (14) pour former la position d'ouverture et que la porte (13) est en cours de fermeture.

3. Agencement (10) selon l'une ou plusieurs des revendications précédentes,
**caractérisé en ce qu'**au niveau du logement (15) est placée une plaque de conducteur (27) avec un capteur (28) qui détecte si la pince de tube est introduite ou non.

4. Agencement (10) selon les revendications 1 à 3,
**caractérisé en ce que** pour garder la pompe à perfusion (14) lorsqu'elle n'est pas utilisée, une sécurité magnétique (29,30) est prévue qui maintient la porte (13) fermée tandis que la butée (25) est placée sur la targette de verrouillage (20).

5. Agencement (10) selon les revendications 1 à 4,
**caractérisé en ce que** la pince de tube (16) est encliquetable en adaptation de forme dans le logement (15).

6. Agencement (10) selon les revendications 1 à 5,
**caractérisé en ce que** dans le boîtier, des guides de tube horizontaux sont prévus à l'entrée et à la sortie.

7. Agencement (10) selon les revendications 1 à 6,
**caractérisé en ce que** la porte (13) s'étend sur presque toute la largeur du boîtier et est basculable de bas en haut dans la position de fermeture.

8. Agencement (10) selon l'une ou plusieurs des revendications 1 à 7,
**caractérisé en ce que** la pince de tube (16) anti-écoulement libre est positionnée après l'entrée du boîtier et avant l'entraînement de la pompe à perfusion et est composée d'une pièce d'injection en plastique, dont les branches (19) sont entourées par un ressort de friction métallique (21).

9. Agencement (10) selon l'une ou plusieurs des revendications précédentes 1 à 8,
**caractérisé par** un tube à pince (12) en PVC ou sans PVC avec une dureté shore de A 75 à 85 avec un diamètre interne de 3,0 mm et un diamètre externe de 4,10 mm, la force de serrage de la pince de tube (16) étant au moins de 10N et particulièrement de 15N.

10. Agencement (10) selon l'une ou plusieurs des revendications précédentes 1 à 9,
**caractérisé par** une pompe à perfusion (14) volumétrique avec entraînement péristaltique.
